# EUROPEAN PATENT SPECIFICATION

(11) **EP 1 064 406 B1**
(45) Date of publication and mention of the grant of the patent: **04.08.2004**
(21) Application number: 99915651.6
(22) Date of filing: 17.03.1999
(51) Int. Cl.: C12Q 1/68

(54) **SOLUTION PHASE NUCLEIC ACID hybridisation SANDWICH ASSAY**
NUKLEINSÄURE HYBRIDISIERUNG-TESTVERFAHREN IN LÖSUNG
Procédé de détection de type sandwich d'acides nucléiques en solution

(30) Priority: 19.03.1998 GB 9805935
(43) Date of publication of application: 03.01.2001
(73) Proprietor: Novartis AG, 4056 Basel (CH); Novartis-Erfindungen Verwaltungsgesellschaft m.b.H., 1235 Wien (AT)
(72) Inventor: ABEL, Andreas, CH-4102 Binningen (CH); BECK, James, Joseph, Cary, NC 27513 (US); EHRAT, Markus, CH-4312 Magden (CH); OROSZLAN, Peter, CH-4053 Basel (CH)
(74) Representative: Dannappel, Hans-Jochen, Dr.
(86) International application number: PCT/EP1999/001782
(87) International publication number: WO 1999/047705

(56) References cited:
- EP-A- 0 423 839
- WO-A-91/14788
- WO-A-93/06241
- WO-A-95/16055
- WO-A-96/35940
- WO-A-98/02580
- PIUNNO P A E ET AL: "FIBER-OPTIC DNA SENSOR FOR FLUOROMETRIC NUCLEIC ACID DETERMINATION" ANALYTICAL CHEMISTRY, vol. 67, no. 15, 1 August 1995 (1995-08-01), pages 2635-2643, XP000520162 ISSN: 0003-2700
- PILEVAR S ET AL: "TAPERED OPTICAL FIBER SENSOR USING NEAR-INFRARED FLUOROPHORES TO ASSAY HYBRIDIZATION" ANALYTICAL CHEMISTRY, vol. 70, no. 10, 15 March 1998 (1998-03-15), pages 2031-2037, XP000766181 ISSN: 0003-2700

## Description

This invention relates to the non-isotopic quantitation of polynucleotides, particularly sequence specific recognition and quantitation by means of optical affinity sensor technology.

A method has been developed for quantifying nucleic acids directly in clinical samples of cytomegalovirus (CMV), hepatitis B (HBV), hepatitis C (HCV) and immunodeficiency virus (HIV) using branched DNA (bDNA) technology (Dewar *et al* (1994) *J. Infect. Dis.* **170,** 1172-1 179; US 4,868,105). The method, which uses large-branched oligonucleotides for signal amplification and enzymatic signal generation, is capable of detecting a few thousand genomes (physiological concentrations) of a given virus. A solution phase nucleic acid sandwich assay using bDNA technology is described in US 4,868,105.

A solution phase nucleic acid sandwich assay makes use of the minimum of a set of capture probes and a set of label probes to detect the target nucleic acid, by immobilising the label probe (in a capture probe/target nucleic acid/label probe sandwich) on a surface, so allowing separation of bound and unbound label and detection/quantitation of the bound (or unbound) label and hence target nucleic acid. The capture and label probes comprise nucleic acids with regions complementary to the target nucleic acid. The label probe may comprise bDNA, as described in US 4,868,105.

The use of a signal amplification procedure allows sensitive detection and quantification, but has the disadvantage that quantification may not be accurate, particularly if the target polynucleotide to be measured is obtained in a complex medium such as blood, tissue or plant extracts. Molecules present in the sample may interfere with the signal amplification and thereby reduce the reproducibility and hence suitability for use as a quantitative method. Sample preparation steps to reduce the level of interfering contaminants may be performed but these may be time consuming and introduce further sources of variation.

Thus disadvantages of signal amplification strategies, especially bDNA, are i) the necessity of a further amplification step, ii) the cumbersome chemistry, possibly associated with fairly high NSB/non-specific hybridisation (NSH) by the last couple of assay steps, i.e. hybridisation of bDNA to the surface bound target DNA complex and hybridisation of the label probe (bulky enzyme label) to the bDNA iii) slow, sluggish binding kinetics therefore low efficacy of bDNA step itself and iv) extended (lengthened) assay procedure.

Polynucleotides have a tendency to strongly associate with other charged macromolecules. like proteins. As a rule of thumb, the larger/bulkier the nucleotide, the stronger this association may be. This makes performing the whole bDNA assay in one step impossible as the bDNA may bind to macromolecules in the sample medium. Binding of various species non-specifically to the compiex in solution phase or on the surface may capture an overproportional amount of bDNA during the later phase of the assay. All of these may result in elevated measurement variability, especially when dealing with complex sample media.

With the above method it is also necessary to separate the label bound to the target polynucleotide from unbound label. This may introduce further variability into the measurements. If the label is detected by fluorescence, chemiluminescence or absorbance, the optical opacity of the sample medium may impair detection of label remaining in the sample medium and introduce further variability.

Other methods of detecting a polynucleotide at low concentrations make use of an amplification method such as the polymerase chain reaction to increase the number of copies of the target polynucleotide prior to use of a detection method. Diagnostic systems operating with target amplification approaches, such as PCR, may suffer from other quantitation problems, i.e. introducing error during the amplification procedure. Mutations in the target polynucleotide sequence can also have a several hundredfold effect on detection efficiency (Urdea (1994) *Biotechnology* **12**, 926-928).

There is therefore a need for a method of detecting a target polynucleotide at very low concentrations that does not require an amplification step, and that is less prone to interference by components of the sample material.

### Summary of the invention

This need can be satisfied by a method based on the detection of luminescence excited in the evanescent field of an optical waveguide.

The method of detecting a target polynucleotide uses a solution phase nucleic acid sandwich assay, wherein the solid support is an optical planar waveguide, and the label is detected by measuring the luminescence that is excited in the evanescent field of the waveguide or by measuring the luminescence that is generated in the near-field of the waveguide.

The phenomenon of the evanescent field can be described using the example of a planar optical waveguide. If a lightwave is coupled into a pianar optical waveguide that is surrounded by media of lower refractive indices, it is confined by near-total reflection at the boundaries of the waveguiding layer. In the simplest case, a planar optical waveguide consists of a three-layer system: substrate, waveguiding layer, superstrate (or samples to be investigated), the waveguiding layer having the highest refractive index. Additional intermediate layers can further improve the action of the planar optical waveguide.

In that arrangement, a fraction of the electromagnetic energy enters the media of lower refractive index. That portion is termed the evanescent (decaying) field. The strength of the evanescent field depends to a very great extent upon the thickness of the waveguiding layer itself and upon the ratio of the refractive indices of the waveguiding layer and of the media surrounding it. In the case of thin waveguides i.e. layer thicknesses that are the same as or smaller than the wavelength that is to be guided, discrete modes of the guided light can be distinguished.

Using an evanescent field it is possible, for example, to excite luminescence in media of relatively low refractive index, and to excite that luminescence in the immediate vicinity of the waveguiding region only. That principle is known as evanescent luminescence excitation.

Evanescent luminescence excitation is of use in analysis, since the excitation is limited to the immediate vicinity of the waveguiding layer. Thus any luminescent molecules not in the immediate vicinity of the waveguiding layer will not be detected and do not need to be removed in order to measure luminescent molecules that have been bound, for example by a biochemical interaction, at the surface of the waveguide.

Methods and apparatus for determining the evanescently excited luminescence of antibodies or antigens labelled with luminescent dyes are known and are described in, for example, US-A-4,582,809. The arrangement claimed therein uses an optical fibre for evanescent luminescence excitation. Such optical fibres have, typically, a diameter of up to a millimetre and guide a large number of modes when laser light is coupled into them. The evanescently excited luminescence can be measured easily only by means of the portion coupled back into the fibres. The apparatus is relatively large and comparatively large sample volumes are required. The sensitivity is limited by the relatively weak evanescent field intensities associated with highly multimode waveguides. The sensitivity is further limited by the endface output-coupling: as excitation and luminescence radiation run colinearly and have to be separated by beam splitters and filters (e.g. cut-off filters or band - pass filters), the discrimination characteristics of the filter unit limits the detection sensitivity.

Photometric instruments for determining the luminescence of biosensors under evanescent excitation conditions using planar optical waveguides are likewise known and are described, for example, in WO 90/06503, which uses planar waveguides 160nm to 1000nm thick. The excitation wave is coupled in without grating couplers.

Various attempts have been made to increase the sensitivity of evanescently excited luminescence and to produce integrated optical sensors. These have included improvements in the manufacture of planar waveguides of high uniform optical quality and reproducible constant layer thicknesses, and improvements in the nature of the coupling of the lightwave into the waveguiding layer, including the use of gratings (Chemical, Biochemical and Environmental Fiber Sensors V (1994), *Proc. SPIE,* **2068**, 313-325). The use of gratings in combination with luminescence is described in US-A-5,081,012 and WO 95/33198. In US-A-5,081,012 excitation light is coupled into the waveguiding film of 200nm - 1000 nm thickness and is propagated, through that part to be used as the sensing region, up to a second, reflective, grating, with the result that the coupled-in light wave has to pass at least twice through the sensor region between the gratings. That is supposed to increase sensitivity. However, a disadvantage is that reflection can lead to an increased scattering of excitation light and an increase in background radiation intensity. In WO 95/33198 a first grating is used for coupling excitation light into the waveguide, and a second separate grating for outcoupling that amount of luminescence that is excited along the propagated excitation mode and has coupled back into the waveguide.

Besides using the evanescent field that is associated with the excitation light propagated in the waveguiding layer for excitation of luminescent molecules close to the waveguide surface, it is also possible to excite luminophores in the near-field of the waveguide using classical configurations of transillumination or surface illumination. The analyte located on the surface of the wave-guiding layer is directly illuminated with the excitation light either through the support of the planar waveguide or through radiation opposite to the wave-guiding layer. It should be understood that the near-field is defined by the penetration depth of the evanescent field of the guided luminescence. In this case the amount of excitation light propagated in the waveguide is negligible. From the amount of luminescence originating from luminophores in the near-field of the waveguide, however, a significant amount is coupled into the waveguide and can be passed to an optical detector using an outcoupling diffractive grating. Due to the high geometrical spatial selectivity of the backcoupling mechanism with this configuration, resulting in very low background signals, high sensitivity can be achieved.

Improvements have also been made to allow the detection of evanescently excited luminescence in multiple samples simultaneously or in rapid succession. WO 94/27137 proposes, for example, an apparatus and a method for carrying out immunoassays using evanescently excited fluorescence.

In WO 92/19976 an arrangement comprising a plurality of integrated measuring strips for detecting a complex signal, for example the detection of an odour by an artificial nose, is described.

In WO 96/35940, a sensor platform based on at least two planar, separate, inorganic, dielectric waveguiding regions on a common substrate is described, which platform is suitable for the parallel evanescent excitation and detection of the luminescence of identical or different analytes. Those separate waveguiding regions may each have one or more coupling gratings. A substantial advantage of that sensor platform is that, for example, several sample solutions can be analysed simultaneously with a high degree of sensitivity. As a consequence of the real time sensing capability, no washing or cleaning steps between individual measurements are required, with the result that a high sample throughput per unit time is achieved. In addition to the analysis of a plurality of sample solutions simultaneously, it is also possible for one sample solution to be tested for several of its analytes simultaneously or in succession on one sensor platform. That is advantageous particularly in the case of blood or serum testing which can thus be carried out especially quickly and economically. When several sample solution are analysed simultaneously, the separate waveguiding regions prevent cross-talk between luminescent signals from different samples. A high degree of selectivity and low error rates are achieved with this method.

The individual separate waveguiding regions can be selectively addressed optically, chemically or fluidically.

Especially suitable is a sensor platform having physically or optically separate planar waveguiding regions in which only one mode or a small number of modes are guided. Such a sensor platform is described in WO 95/33197. It is distinguished by an especially high degree of sensitivity and an extremely small structure. Using the waveguiding film parameters as described in WO 95/33197, the attenuation of the guided lightwave can be less than 3dB/cm, thereby resulting in a long distance guided beam and a low scattering of the guided wave into the medium surrounding it. Such a sensor platform may be known as a "chip". As a rule, the same degree of sensitivity is not achieved with multimodal waveguides of planar construction.

The use of evanescently excited fluorescence based methods for the detection and quantitation of polynucleotides is mentioned in WO 95/33198, WO 96/35940 and WO 95/33197 but methods to optimise the sensitivity of detection are not described. WO 95/16055 describes a bulk fluorescence excitation method, wherein sensibility is enhanced by using an amplifier bond to the target, to which are bond multiple label probes, and keeping specific binding conditions for capture, capture extender, label extender and label probes.

This invention exploits the sensitivity of detection of evanescently excited luminescence in a method of detecting polynucleotides especially/ at low concentrations. The method makes use of multiple fluorescently labelled polynucleotides (label extender probes) that are bound to or bind a target polynucleotide that is captured (ie denatured and immobilised) on the surface of an optical planar waveguide. The target polynucleotide is captured by binding to one or a series of capture extender probes which bind to multiple copies of a single species of capture probe that are bound to the waveguide. Alternatively the capture extender probe (if no capture probe is used) or capture probe may further comprise a ligand which is capable of binding to a receptor which is bound to the surface of the waveguide. This is possible as it will not place the label too far from the surface of the waveguide, since the evanescent decays exponentially with distance from the surface. The bound label is then detected in the evanescent field. The bound label is proportional to the amount of target polynucleotide present in the assay sample, and the amount of target polynucleotide may be quantified by reference to samples containing known amounts of the target polynucleotide or using a master curve prepared using any polynucleotide with the same sequence of known concentration and purity. Quantitation is also possible using internal standards or a two or multi wavelength approach, i.e. energy transfer or two label coincidence approach. The internal standard(s) must not be the target polynucleotide; they can be any sequence designed to fulfil criteria to be used as an internal standard.

The method may lead to a higher efficiency of the detection cascade compared to the bDNA approach (i.e. a much higher overall yield of captured label), and therefore an equal or higher sensitivity, allowing detection of < 50 target sequences, which may be equivalent to physiological concentration of a relevant biological target sample (e.g. HIV viral mRNA).

By "optical planar waveguide" is meant a waveguide consisting of substantially planar support and a thin transparent layer on one surface having a higher refractive index than the support, both the support and the thin waveguiding layer on its surface being transparent at least in the spectral region between the excitation and the emission wavelength to be detected. In contact with the waveguiding layer an optical coupling element, preferably a diffractive optical element (DOE), for incoupling of excitation light, is modulated. Preferably the DOE is a relief grating. The grating may be first generated in the support layer, for example by photolithographic methods, as described in Proc. SPIE, 2068 (1993) 313 - 325, and then be transferred into the waveguiding layer, upon deposition of the waveguiding layer, or may be generated in the deposited waveguiding layer. More preferably, in the waveguiding layer are modulated two separate gratings, one used as the incoupling grating and the other fr outcoupling of excited emission light. Grating constants and dimensions are described in WO 95/33198, EP-A-0 759 159 and WO 96/35940. Planar waveguide technology is well known in the art, for example as described in the above references. Transparency in this and the following context means transparency in the spectral region from the excitation to the emission wavelength to be detected.

The method of the invention has the advantage that separation of bound and unbound label is not necessarily required as only bound label is detected. The optical opacity of the sample does not significantly affect the detection of bound label. The threefold selectivity incorporated, i.e. the evanescent field for spatial resolution, biochemical recognition for chemical selectivity and fluorescence labelling for enhanced detection selectivity and sensitivity, has been found to allow the measurement of minute amounts of analytes in complex sample media, such as blood, tissue or plant extracts without tedious sample preparation.

It will be appreciated that the method of the invention may use a method of detecting a target polynucleotide using a solution phase nucleic acid sandwich assay which may be similar to that as described in US 4,868,105, as discussed above, except that the solid support is an optical planar waveguide, the bound label may or may not be separated from unbound label prior to measurement of bound label and the labei is detected by measuring the luminescence that is excited in the evanescent field of the waveguide, or by measuring the luminescence, that is generated in the near field of the waveguide.

The two or three hybridisation interactions needed in order to bring the fluorescent label into the evanescent field have been found to render the method of the invention more selective than previously described methods of polynucleotide detection utilising planar waveguides which envisage a single hybridisation interaction. The multiple capture and label extender probes that may bind to a target polynucleotide have been found to render the method more sensitive than previously described methods.

It will also be appreciated that recoating of the waveguide with capture probes may not be necessary to detect different target polynucleotides using a method of the invention as the target polynucleotide does not hybridise directly to the capture probe, in contrast to previously described methods (for example in WO 95/33198 and WO 96/35940) where the target polynucleotide does hybridise directly to the polynucleotide bound to the waveguide, where recoating with a different polynucleotide may be required. The sequence of the capture extender probe may be varied so that different capture extender probes are able to bind to a common capture probe but different target polynucleotides.

A first aspect of the invention comprises a method of detecting a target polynucleotide employing two sets of reagents, a labelling set and a capture set, and wherein an optical planar waveguide is used, said method comprising
1) providing the labelling set of reagents, comprising
   a) a plurality of label probes each comprising a label portion and a nucleic acid region (which is preferably single-stranded) having same or different sequences L1 (preferably between about 5 and 100 nucleotides (nt) long; more preferably between about 8 and 100 nt long) which are complementary to same or different sequences of the target polynucleotide, and optionally second sequences between the label portion and sequences L1, which are neither complementary to a sequence of the target polynucleotide nor to the capture extender or capture probes and which are less than about 500 nt long, and the label portion comprising a label which provides a signal which is detectable by luminescence, including evanescently excited luminescence, or
   b) a plurality of (b1) label extender probes each comprising a label binding nucleic acid sequence L2 and nucleic acid sequences L1, wherein the sequencies L1 are complementary to nucleic acid sequences of the target polynucleotide and the sequence L2 is complementary to the nucleic acid sequence L3 of a label probe, and optionally (b2) a label probe comprising a nucleic acid sequence L3, which is complememtary to the the sequence of L2, and a label portion comprising a label which provides a signal which is detectable by luminescence, including evanescently excited luminescence, and
2) providing the capturing set of reagents, comprising
   c) a plurality of capture probes comprising same or different nucleic acid sequences D1 complementary to nucleic acid sequences of the target polynucleotide, wherein the nucleic acid sequences D1 (preferably between about 10 and 100 nt long) is not complementary to sequencies L1, L2 and L3 comprised in the label probe and the label extender probe and wherein the capture probe is bound or capable of being specifically bound to the surface the wave guiding layer of a waveguide, either directly or via linking groups; or
   d) a plurality of (d1) capture extender probes each comprising a nucleic acid region (preferably single-stranded) having a sequence Cl (preferably between about 10 and 100 nt long) which is complementary to a sequence of the target polynucleotide and optionally a second region not being complementary to a sequence of the target polynucleotide and preferably being less than about 500 nt long, and further comprising a capture probe recognition nucleic acid sequence C2, and (d2) a capture probe and wherein the capture probe is bound or capable of being specifically bound to the surface the wave guiding layer of a waveguide, either directly or via linking groups, comprising nucleic acid sequences D2 complemetary to sequence C2, wherein sequences L1 and C1 are non-identical, non complementary sequences;
3) combining in an aqueous medium under binding conditions for complementary sequences, a sample in which the presence of the target polynucleotide is to be determined (which may have been treated so that the target polynucleotide is present in single-stranded form), with
   a) compositions 1a) and 2c) or 1a) and 2d), or (b) compositions 1b) and 2c) or 1b) and 2d), or (c) compositions 1a), 1b) and 2c) or 1a), 1b) and 2d), or (d) compositions 1a), 2c) and 2d) or 1b), 2c) and 2d), ), or (e) compositions 1a), 1b), 2c) and 2d), so that complexes are formed;
4) binding the complexes of step 3 to the waveguide via a plurality of copies of the capture probe (the capture probe may be bound to the waveguide before binding to the complexes of step 3 or may be bound to the waveguide after binding to the complexes of step 3);
5) if the composition 1b) does not comprise a label probe and a label was not included in step 3 to label said complexes, then combining the bound complex of step 4 with the label;
6) detecting label bound to the target polynucleotide by measuring the luminescence that is excited in the evanescent field of the waveguide, or by measuring the luminescence that is generated in the near field of the waveguide,
whereby at least two label probes or label extenders, capture probes or capture extender probes, are used having different nucleic sequences which are complementary to different target nucleic acid sequences.

It is preferred that each of the sequences L1 is complementary to a respective physically distinct, non-overlapping region of said target polynucleotide; and it is preferred that each of the sequences L1 and C1 are non-identical, non complementary sequences that are preferably each complementary to physically distinct, non-overlapping sequences of said target polynucleotide.

Plurality means in the context of this invention, that there may be used up to 100, preferably 5 to 80, more preferably 5 to 50, most preferably 5 to 40, and particularly preferred 10 to 30 having different nucleic sequences.

Preferably, the luminescence is coupled into the waveguide, guided and outcoupled using an optical coupling element.

It is preferred that C1, C2, L1 and D1 are not identical to each other and that C1, L1 and D1 are not complementary to each other. It is further preferred that if the label extender comprises a binding site for the label and that binding site comprises a single stranded nucleic acid sequence (L2) and the label comprises a single stranded nucleic acid sequence (L3) complementary to L2, that L3 is not complementary to D1.

As a preferred embodiment of the invention in this context the luminescence excited in the evanescent field of the waveguide is detected.

Further embodiments include the detection of luminescence. chemoluminescence, bioluminescence or electroluminescence generated in the near-field of the waveguide. Preferably the said luminescence is coupled into the waveguide, guided and outcoupled using an optical coupling element.

A preferred optical coupling element for coupling excitation light into, or guided luminescence out of, the waveguide are gratings modulated in the waveguiding layer ot in the support material.

It will be appreciated that the following points may be relevant to more than one aspect of the invention as herein described.

It is preferred that the optical planar waveguide is an optical planar waveguide on a continuous substrate. It is further preferred that the substrate is transparent glass, quartz or a transparent thermoplastic plastics material like polycarbonate. There may further be a transparent intermediate layer between the substrate and the waveguiding layer with a refractive index lower than that of the waveguiding layer, preferably also lower than the refractive index of the substrate.

The refractive index of the waveguide layer should be greater than that of the substrate and of any intermediate layers that are used. The planar, transparent waveguiding layer preferably consists of a material having a refractive index greater than 1.8, still more preferably greater than 2.0. Suitable materials include, for example, inorganic materials, especially inorganic metal oxides, such as TiO₂, ZnO, Nb₂O₅, Ta₂O₅, HfO₂, or ZrO₂. TiO₂ or Ta₂O₅ are preferred. It is preferred that combinations of inorganic metal oxides are not used.

The thickness of the waveguiding layer is preferably from 40 to 1000 nm, more preferably from 40 to 300 nm and most preferably from 70 to 160 nm.

The modulation depth of the gratings is preferably from 3 to 60 nm, more preferably from 3 to 30 nm. The ratio of the modulation depth to the thickness of the waveguiding layer is preferably equal to or less than 0.5 and more preferably equal to or less than 0.2.

The gratings may be in the form of optical diffractive gratings, preferably in the form of relief gratings. The relief structure may have various forms, for example sinusoidal, rectangular or sawtooth structures.

The grating structure can be produced on the substrate and then transferred to the waveguiding layer, where the grating structure then reproduces itself, or the grating is produced in the waveguiding layer itself.

The grating period may be from 200 nm to 1000 nm, selected to allow light to be coupled in or out of the waveguide in the first diffraction order.

The waveguiding layers preferably guide only 1, 2, 3 or 4 modes and are more preferably monomodal waveguides.

It will be appreciated that the label extender probes, capture extender probes and capture probe may comprise single-stranded nucleic acid regions.

It will further be appreciated that by "nucleic acid" is included DNA, RNA, PNA (peptide nucleic acid) or other modified oligonucleotides such as 2'-O-methyl or P=S (phosphorothioate) analogues Modified oligonucleotides and nucleic acid analogues may be useful in optimising the assay for sensitivity and specificity. Additionally, modified oligonucleotides can not be digested by nucleases and are therefore more stable in real samples like blood serum or plant extracts. Optimisation may be achieved by carrying out kinetic analyses, for example using computer aided assay design.

It will be appreciated that the steps may not be performed in the order listed, although it is preferred if the steps are performed in the order listed. In particular, it will be appreciated that the order in which the target polynucleotide, label extender probe, capture extender probe, capture probe and label (if comprised in the label extender probe) bind is not critical to the operation of the invention. It is preferred that the target polynucleotide binds to the label extender probe and capture extender probe before binding to the capture probe (i.e. that a "preincubation" step is performed). This may optimise the signal and minimise the background.

The hybridisation assay can be performed in any possible order and combination, depending on the specific assay target (ss or ds; DNA, RNA) and whether sensitivity or specificity is most important in a particular assay. The two extremes are:
1) A method in which the whole assay is performed in one step *in situ,* for example in a well, where the solution contacts the planar waveguide (PWG) surface with an immobilised receptor or in a separate preincubation chamber. The immobilised receptor is capable of binding the capture extender and therefore is capable of binding the whole sandwich which is formed in solution, including the label.
2) Alternatively, simultaneous hybridisation steps are performed one after the other directly on the surface of the PWG (i.e. a sequential process). Any variation between these extremes may be adopted.

Thus an aspect of the invention is a method according to the first embodiment described above wherein steps 3 and 4 are combined, such that a sample in which the presence of the target polynucleotide is to be determined is combined with the label extender probes and the capture extender probes and the capture probe optionally bound to said planar waveguide, so that (label extender probe) + (target polynucleotide) + (capture extender probe) + (capture probe) complexes are formed which are bound to said planar waveguide via the capture probe. It is preferred that the capture probe is immobilised on the waveguide surface, but it will be understood that more complex multiple sandwich arrangements are possible.

The solution in which assay steps are performed is an aqueous solution. It may be any suitable buffer solution, for example 75 mM sodium citrate, 750 mM sodium chloride, 0.1% Tergitol NP-40 (Tergitol is a trademark for a range of wetting agents), adjusted to pH 7.0.

Suitable buffer components may include:

| | |
|---|---|
| 0 - 2 M | sodium citrate |
| 0 - 2 M | sodium chloride |
| 0 - 2% | Tergitol® NP-40 |
| 0 - 2 % w/v | Tween-20® or 80 or another detergent |
| 0 - 2 M | Tris-HCl, pH 6 - 8 |
| 0-2% | Bovine Serum Albumin (BSA) |
| 0 - 500 mM | EDTA |
| 0 - 2% w/v | tartrazine |

By "complementary sequence" is meant a sequence that is able to hybridise with a polynucleotide sequence under conditions used in the assay. By "hybridise" is meant that the sequences are able to form a stable structure together, which can be detected by hybridisation assays well known in the art. These conditions may be such that hybridisation will occur only if the sequences are at least 60% inversely identical, more preferable at least 80% inversely identical, still more preferably at least 90% inversely identical and most preferably at least 95% inversely identical. By "% inversely identical" is meant the percentage of bases (in the region being considered) that are theoretically able to form base pairs when single stranded sequences are aligned with one strand 5'- 3' and the other 3'-5'. The hybridisation steps may be carried out at between 0 °C and 90 °C in the buffer described above; preferably they are carried out at 53 °C.

It will be appreciated that the target polynucleotide or the signal may be amplified prior to detection but that amplification is not essential.

It will be appreciated that the polynucleotide may be detected at extremely low concentration. By this is meant that less than 10⁴ copies of a target polynucleotide can be detected, preferably less than 10³ copies, still more preferably 10² copies or less. Sample volumes may be between 10 nl and 10 ml. It has been shown to be possible to detect less than 1000 equivalents of the genomic DNA in 10 µL of asample, as described in the Examples.

It is not essential not to remove the sample and unbound label prior to capturing the specific, target related signal. In cases of extremely high sensitivity, it can happen that the "bulk" signal representing labels resident temporarily in the close proximity of the surface (in the evanescent field) mask the specific signal due to the high label concentration compared to the target level used in the experiment. In such cases, it is advantageous to remove unbound labels (by washing the cell) prior to signal recording.

The target polynucleotide may be DNA or RNA, ss or ds. For example, it may be a mRNA, a viral DNA or a genomic sequence from a pathogenic organism. It may be any gene of interest, for example for experimental or diagnostic purposes. It will be appreciated that the target polynucleotide may be derived from a plant pathogen or an animal or human pathogen. An example of a plant pathogen from which a target polynucleotide may be derived is *Pseudocercosporella herpotrichoides.* A preferred option, important in clinical diagnostics is that the pathogen is a virus. It is particularly preferred if the virus is CMV (cytomegalovirus), HBV (hepatitis B virus), HCV (hepatitis C virus) or HIV (human immunodeficiency virus). This assay may, for example, be used to identify and differentiate the five genotypes of the Hepatitis C virus (Cha *et al* (1992) *PNAS* **89**, 7144-7148). Other targets are marker genes or drug responsive messages ie mRNAs whose abundance changes in response to a particular drug.

It will also be appreciated that the target polynucleotide may be a polynucleotide comprising a sequence that is associated with a disease. For example it may comprise a mutation that is statistically linked with cancer or with a particular form of cancer. Examples of forms of cancer which have been linked with particular mutations include prostate cancer, breast cancer, colon cancer and leukaemias. An example of a mutation that may be linked with disease is the cystic fibrosis gene. Mutations in enzymes identified by pharmaco- and toxicogenetics may also be detected, which may include a P450 enzyme.

The combination of planar waveguide technology with DNA/RNA hybridisation allows the detection of disease markers such as single stranded DNA, RNA or denatured double stranded DNA. The DNA target may be, for example, a DNA fragment, viral DNA or genomic DNA.

If the polynucleotide is a ds polynucleotide or triple helix, it should be denatured prior to detection. Denaturation may be carried out by any suitable means, such as heat or alteration of ionic strength or pH. Alternatively, singie stranded copies may be made by asymmetric PCR. It is preferred that single stranded copies are prepared by denaturation rather than asymmetric PCR as use of a PCR step may introduce variability, as described above. It will be appreciated that denaturation may be performed after combining the sample containing the target polynucleotide with the assay reagents. Preferably it is performed after such combination. This has the advantage that the opportunities for renaturation of the target polynucleotide before contact with the assay reagents are reduced. Denaturation may conveniently be performed by heating to 95 °C for 5 minutes.

The set of label extender probes for detecting a given target polynucleotide comprises at least two types of probe and may comprise up to 30 types of probe. It is preferred if the set comprises between 5 and 30, more preferably between 10 and 25 and most preferably between 15 and 25. types of probe. It will be appreciated that (i) the optimum size of the set may be target and assay dependent, and (ii) the amplification achieved is proportional to the number of label extender probes used. Each label extender probe comprises a single-stranded nucleic acid region and a label portion, the nucleic acid region having a sequence L1 which is complementary to a sequence of the target polynucleotide, and the label portion comprising a label which provides a signal detectable by luminescence, for example evanescently excited luminescence, or a binding site for said label, wherein each of said sequences L1 is complementary to physically distinct, non-overlapping regions of said target polynucleotide. Each label extender probe may further comprise a second nucleic acid region which is not complementary to a sequence of the target polynucleotide and which is less than about 500 nt.

The binding site for the label may comprise a single-stranded polynucleotide sequence. It may be a sequence (L2) that is complementary to a sequence (L3) of a polynucleotide to which is bound one or more label molecules. This second sequence will be selected so as not to be complementary to a sequence of the target polynucleotide sequence or any other sequence likely to be encountered in the sample. It will be appreciated that if the binding site for the label comprises a single-stranded polynucleotide sequence that the label extender probe may correspond to a "chain extender" as used in some sandwich assays known in the art and the label corresponds to a "label probe".

The binding site and label may alternatively respectively comprise molecules with high specificity and affinity for each other, for example biotin/avidin or streptavidin. Thus the binding site may comprise biotin and the label may comprise avidin, or *vice versa*. Alternatively, the label may be bound directly to the label extender probe.

The region L1 will generally be between 5 and 5000 nucleotides, preferably between 10 and 500 nucleotides, still more preferably between 15 and 50 nucleotides in length. It will be appreciated that the region L1 may be joined to a second nucleic acid region which is not complementary to a sequence of the target polynucleotide at the 3' or 5' end.

The complementary sequences will be chosen so as to leave sequences of the target polynucleotide for the capture extender probe to bind to. Usually sequences of at least 25 nucleotides will be left available. The sequences complementary to the label extender probes may be separated or substantially contiguous. They may be alternated with sequences that are complementary to the sequences of the capture extender probes. It is preferred that the sequences complementary to the label and capture extender probes are spread throughout the target sequence and are alternated.

The sequences and number of capture extender probes which are complementary to the target polynucleotide may be selected on the basis of particular criteria. For example, if it is desired to quantify the amount of polynucleotide with a particular mutation then sequences may be chosen that only bind to polynucleotide molecules with that particular mutation under the conditions of the assay.

The label extender probe may be conveniently prepared by known methods of oligonucleotide synthesis or by cloning and may be modified as appropriate for labelling. It is preferred if the sequences are prepared by synthesis. By providing for a terminal group which has a convenient functionality, labelled molecules may be joined through the functional group. Examples include carboxy, thio, amine or hydrazine.

The label comprises a luminescent compound. Preferably the compound has a luminescence wavelength in the range of 330 nm to 1000 nm. Examples include, but are not limited to: rhodamines, phycoerythrin, umbelliferone, luminol, Texas red, fluorescein derivatives, coumarin derivatives, distyryl biphenyls, stilbene derivatives, phthalocyanines, naphthalocyanines, polypyridyl/ruthenium complexes, such as tris(2,2'-bipyridyl)ruthenium chloride, tris(1,10-phenanthroline)ruthenium chloride, tris(4,7-diphenyl-1,10-phenanthroline) ruthenium chloride and polypyridyl/phenazine/ruthenium complexes, platinum/porphyrin complexes, such as octaethyl-platinum-porphyrin, long-lived europium and terbium complexes or cyanide. Particularly suitable for analyses in blood or serum are dyes having absorption and emission wavelengths in the range of from 600 to 900 nm.

Dyes, such as fluorescein derivatives, containing functional groups by means of which they can be covalently bonded, such as fluorescein isothiocyanate, are preferred. Functional fluorescent dyes that are commercially available from Biological Detection Systems Inc. for example the mono- and bi- functional Cy5^{TM} dyes *(Clin. Chemistry* (1994) **40(9)**, 1819-1822), are particularly preferred.

Additionally, the longer emission wavelength of red and NIR (near infra red) dyes may be advantageous for certain applications, due to the larger penetration depth of the evanescent field (the longer the wavelength the larger the penetration depth of the evanescent field). Labelling oligonucleotides during the last step of DNA synthesis is favourable, resulting in a higher yield and homogeneity of the labelled oligonucleotide, for example with Cy5^{TM}.

The capture probe may comprise a nucleic acid sequence complementary to the capture probe recognition sequence, wherein the nucleic acid sequence is between about 10 and 100 nt long and is not complementary to sequences comprised in the label extender probe and wherein the capture probe is bound or capable of being specifically bound to a planar waveguide. It may be a polynucleotide of between 10 and 100 nucleotides in length, preferably between 15 and 50 nucleotides, more preferably between 18 and 30 nucleotides in length. The capture probe may have a "universal" sequence, such that it may be used in the detection of several different target polynucleotides. A preferred capture probe sequence is 3'-TTATAGTACTCCAATGCC-5'. This sequence has beneficial stability against exonucleases due to the immobilisation on the 3' end to the surface. The attachment of the capture probe at the 3'-end is beneficial, because the capture probe can not be digested by most exonucleases attacking oligonucleotides at the 3'-end.

The capture probe may be synthesised by known synthesis protocols. It is preferred if it is synthesised on the waveguide "chip" so that it is immobilised by the 3' end. For example, it may be synthesised with an oligonucleotide synthesiser (Applied Biosystems 394B) direct on the waveguide chip silanised with 3-glycidyloxypropyltrimethoxysilane by a modification of a standard process used for oligonucleotide synthesis on particles (for example as described in Gait (1990) *Oligonucleotide synthesis: a practical approach* Oxford University Press, NY). Instead of the standard synthesis, 4-(4,4-dimethoxytrityl)hydroxybutyric acid is used as a stable linking group for anchoring the 3' end of the oligonucleotide to the surface. The surface may be washed with water prior to use in the assay.

Whilst the above method may be used, the following method is preferred:
**a)** PWG-chips are cleaned with CHCl₃
**b)** liquid phase silanisation of the PWG-chips is carried out by treatment with 2 % (v/v) 3-glycidyloxypropyl trimethoxysilane and 0.2 % (v/v) N-ethyldiisopropylamin in o-xylene at elevated temperature (i.e. 50-90 °C) for several hours
**c)** silanised PWG-chips are cleaned in CH₃CN
**d)** PWG-chips are dried in a desiccator under vacuum
***e) Immobilisation of Oligonucleotides***
   The surface bound epoxy rings may react via ring opening with terminal amino or thiol groups of modified oligonucleotides: 100 µl of a solution containing 10 nmol of a thiol or amino terminated capture probe is incubated at 35 °C over night in 100 mM aqueous carbonate buffer, pH 8.2.
**f)** PWG-chips with immobilised capture probes are rinsed with water, dried and stored at -20 °C until used in the PWG setup.

The capture probe may also be anchored on the waveguide by other means. For example, photochemical cross-linking may be used, as described in WO 94/27137. An adhesion-promoting layer may be located between the waveguiding regions and the immobilised capture probe. The thickness of the adhesion-promoting layer is preferably equal to or less than 50 nm, more preferably less than 20 nm and still more preferably less than 10 nm.

The solution containing the capture probe may be applied in a dropwise manner using a multi-pipette head or modified inkjet printing head with piezoelectric actuators. This has the advantage that the method can be carried out rapidly and that very small amounts can be used. If a "universal" capture probe is used, the same solution may be applied to all sensing regions.

The capture probe and subsequent reagents may be delivered to the sensing regions by means of a flow cell. Separation of the regions can be effected mechanically by the use of dividing bars or fluidically in the case of laminar flow.

The selective immobilisation of the specific recognition elements exclusively on the detection regions, either directly or by way of adhesion-promoting layers can, when using a sample cell that covers both the waveguiding and non-waveguiding regions, lead to an increase in the sensitivity of the detection method, since the non-specific binding of the analytes in the regions not used for signal generation is reduced.

It will be appreciated that an adhesion-promoting layer may be applied selectively only in the waveguiding region or may be deactivated in the non-waveguiding regions, for example by means of photochemical activation or the delivery means described above.

In a general sense the capture probe can be immobilised by methods including hydrophobic adsorption or covalent bonding directly to the waveguiding regions or after chemical modification of the surface, for example by silanisation or the application of a polymer layer. In order to promote the immobilisation of the capture probe directly on the waveguide, a thin intermediate layer, for example consisting of SiO₂ (Boksányi *et al* (1976) *Advanc. Colloid interface Sci.* **6**, 95-137, can be applied as an adhesion-promoting layer.

It will be appreciated that hydrophobic attachment is not possible if the capture probe is a polynucleotide. Covalent or ionic attachment is generally preferred, but other receptor/ligand pairs, such as avidin/biotin. antibody-haptens or recognition systems like (His)₆-tagged oligonucleotide to NTA (nitrilotriacetic acid) are also possible.

The adhesion promoter may be a functionalised silane layer or a polymer layer (e.g. polylysine ; M.Schena, D.Shalon, R.Heller, A.Chai, P.O.Brown and R.W. Davis: Parallel human genome analysis: microarray based expression monitoring of 1000 genes, Proc.Natl.Acad.Sci. USA, 93 (1996) 10614-10619) bearing functions (for example, epoxy, -NH₂, -SH, -COOH, -NHS, maleimide), where the function should be activated prior to the attachment of the functionalised capture probe (functions as listed above). In the case of ionic attachments, conditions favouring ionic interactions between the anchoring function of the capture probe (but not the capture sequence or a member of the entire detection cascade) and the adhesion-promoting (also ionic, complementary charged) layer should be used.

If non-covalent attachment, regeneration of the surface can be performed by creating conditions favouring dissociation of the capture probe from the surface.

It may be possible to remove the target polynucleotide, capture extender probe and label extender probe from the waveguiding surface in such a way that the capture probe is undamaged and the waveguide can be used again for detection of the same or different target polynucleotide. For example, low pH or high pH, elevated temperature, organic solvents or chaotropic agents (salts) may be used to dissociate the target polynucleotide, capture extender probe and label extender probe. pH <4 may damage the DNA and is therefore not preferred. High pH, e.g. 10 mM NaOH, or 50% aqueous urea solution can be used to denature the dsDNA at the sensor surface and thereby dissociate the target polynucleotide and capture extender probe from the capture probe.

Capture extender probes each comprise two single-stranded nucleic acid regions, the first nucleic acid region having a sequence C1 between about 10 and 100 nt long which is complementary to a sequence of the target polynucleotide and the second region not being complementary to a sequence of the target polynucleotide and being less than about 500 nt long and further comprising a capture probe recognition sequence C2, wherein sequences L1 and C1 are non-identical, non complementary sequences that are each complementary to physically distinct, non-overlapping sequences of said target polynucleotide.

It will be appreciated that more than one type of target polynucleotide can be detected at once in a sample. Different specificity capture extender probes and label extender probes can be mixed, and the different target polynucleotides detected and quantified separately if labels are used that have different emission wavelengths and can therefore be distinguished in a suitable waveguide system.

It will be appreciated that the ratio of target to capture extender will require optimisation for a particular target polynucleotide and concentration range. This may be done by altering the ratio of capture extender probe to target polynucleotide whilst using a constant target to label extender ratio. The optimisation process will lead to determining the best results in respect to the ratio of specific to non-specific binding (i.e. binding in the absence of the target polynucieotide).

The ratio of target to label extender may similarly be optimised by applying a constant target to capture extender ratio to find the optimum ratio of target to label extender.

It will be appreciated that the weight ratios, or concentrations, of ingredients, for example label extender (LE), capture extender (CE) and label probe (LP) are essentially assay specific (i.e. may depend on the nature of the sample) and target specific and must be optimised for each target.

It is preferred if the concentrations fall within the following ranges:
1) 0.1 to 30, more preferably 0.5 to 20 and most preferably 0.5 to 10 nM of LE.
2) 0.01 to 20, more preferably 0.1 to 10 and most preferably 0.1 to 5 nM of CE
3) 0.1 to 30, more preferably 0.5 to 20 and most preferably 0.5 to 10 nM of LP.

It will be appreciated that the invention encompasses a method of detecting a target polynucleotide using a planar waveguide, wherein the target polynucleotide is immobilised on the waveguide by means of two classes of binding interactions, the first class of binding interaction being with multiple capture extender probes, and the second class of binding interaction being between at least one of said capture extender probes and at least one of multiple capture probes bound, or capable of being bound and subsequently is so bound, to the surface of the optical planar waveguide. It will be appreciated that the multiple capture probes may be identical to each other.

It will be appreciated that the methods of the invention may be performed using a fully automated analysis system, for example the one described in *Sensors and Actuators* (1997) Vol B38-39, 88-95. This system is designed to investigate bioaffinity interactions in real time. The high sensitivity and selectivity of this system, short assay cycles and the need for only minor sample preparation may allow the detection of polynucleotides without the need of target amplification by PCR or using branched DNA for signal amplification. This may dramatically ease and simplify the quantitation of, for example, mRNA, as a marker of infectious diseases.

A further aspect of the invention is the use of a method of the invention for the investigation or diagnosis of a disease or other response of a living organism associated with the target polynucleotide. For example, an organism may respond to a change in its environment, for example a change in temperature, osmotic shock or the concentration of heavy metal ions, by a temporal or spatial change in gene expression. Thus heat or cold shock may induce changes in the expression levels of particular genes. It will be appreciated that expression of recombinant genes may be measured, for example a recombinant gene whose expression is controlled by a heat shock promoter.

A still further aspect of the invention is an optical planar waveguide wherein is bound to the surface of the wave -guiding layer of the planar waveguide
a) a plurality of capture probes as above defined under 2c), or a plurality of capture extender probes and a capture probe as above defined under 2d);
b) a target polynucleotide, bound to the capture probe or the capture extender probe; and
c) a plurality of lable probes as above defined under 1a), bound to the target polynucleotide, or a plurality of a lable probe extender as above defined under 1b), bound to the target polynucleotide, and a lable probe as above defined under 1b), bound to said lable probe extender.

Preferably the capture probe is a polynucleotide 10 to 100 nucleotides in length. Still more preferably the capture probe is covalently attached to the surface of the waveguide as described above and in Example 1.

A further aspect of the invention is a kit of parts comprising a capture probe, capture extender probe, label extender probe and optionally label all as defined above for carrying out the methods of the invention in relation to a particular target polynucleotide.

A still further aspect of the invention is the use of a waveguide in a nucleic acid sandwich assay for detecting a target polynucleotide.

A further aspect is the use of a non-label-amplified sandwich assay in a wave-guide-mediated polynucleotide detection assay. By "non-label-amplified sandwich assay" is meant a nucleic acid sandwich assay in which bDNA is not used.

A further aspect of the invention is a sandwich assay for detecting a target polynucleotide using an optical planar waveguide.

A still further aspect of the invention is a composition comprising an optical planar waveguide, wherein to the surface of the waveguiding layer is bound directly or indirectly a capture probe or capture extender/capture probes, to said capture or capture extender/capture probe is bound the target polynucleotide, to said target is bound a label extender probe and, if the label extender probe comprises a binding site for a label, said label, wherein all components are as defined in earlier aspects of the invention.

The invention will now be described in further detail by reference to the following figures and examples:

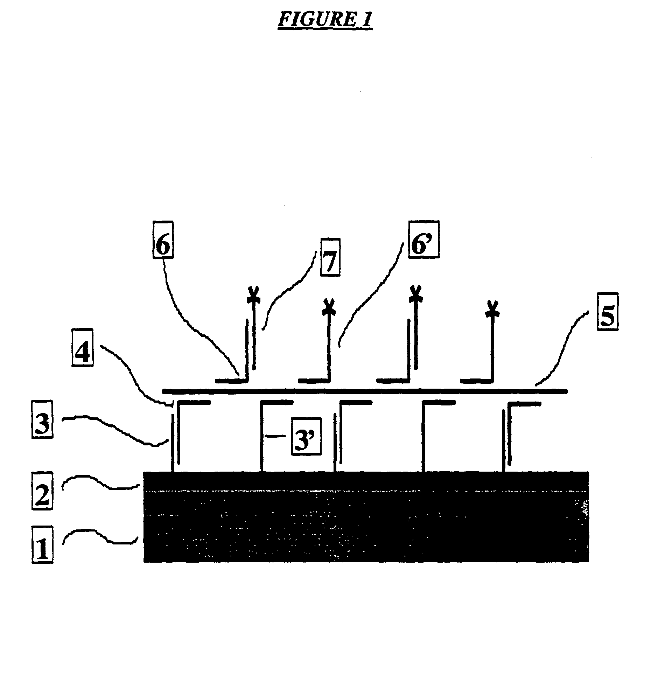

Figure 1 illustrates one possibility of a planar waveguide assay [ named Multiple Oligonucleotide Hybridisation Assay - (MOHA)]. The planar waveguide comprises a transparent support 1 and a wave guiding layer 2. On the surface of the waveguide layer is bound a capture probes 3 with nucleic acid sequence D2, to which is bound via sequence C2 a plurality of capture extender probes 4 having a plurality of different nucleic acid sequences C1. The target polynucleotide 5 is bound to the sequences C1. To the target polynucleotide is bound a plurality of label extender probes 6. having nucleic acid sequences L1 and L2. To said label extender probes are bound via its nucleic acid sequences L3 label probes 7, having nucleic acid sequences L3. In another example, the plurality of capture probes 3' can be bound directly to the target with a nucleic acid sequence D1. In another variant, a plurality of label probes 6' can be bound directly to the target via a nucleic acid sequences complementary to the selected target sequences.

### Example 1: Assay for Pseudocercosporella herpotrichoides: sequence specific detection of the ITS region

The internal transcribed spacer (ITS) region of *Pseudocercosporella herpotrichoides,* the causative agent of eyespot disease in wheat, has been sequenced and used as an unique target gene in a PCR-based diagnostics project. Since the ITS sequence is known, the development of oligonucleotide probes is fairly straightforward. In addition, there are eyespot-infected wheat extracts available with varying levels of infection characterised by the diagnostics project mentioned above. For these reasons, the eyespot ITS region represented a good target for a model planar waveguide based DNA assay. Table 1 shows the molecular weight and the number of basepairs of the genomic DNA, and the PCR amplified ITS fragment of *Pseudocercosporella herpotrichoides.* These targets are all double stranded DNAs, which have to be denatured prior to use. For the optimisation of the assay conditions a single stranded ITS fragment was prepared by asymmetric PCR by using a 20-fold excess of the primer which amplifies the sequence of interest.

**Table 1:**

| double stranded target DNA molecules of different sizes from *Pseudocercosporella herpotrichoides* | | |
|---|---|---|
| target | number of base pairs | molecular weight (Da) |
| ssITS fragment | 627 b | 2.07 x 10⁵ |
| ds ITS fragment | 627 bp | 4.14 x 10⁵ |
| Plasmid vector | 4559 bp | 3.01 x 10⁶ |
| Genomic DNA | 40 Mbp | 2.64 x 10¹⁰ |

The target single stranded ITS sequence is described below. There are also indicated the regions selected for the 20 label extenders (LE) and 5 capture extenders (CE).

1 TCCGTAGGTGAACCTGCGGAAGGATCATTAATAGAGCAATGGATAGACAG 50
51 CGCCCCGGGAGAAATCCTGGGGGCCACCCTACTTCGGTAAGGTTTAGAGT 100
101 CGTCGGCCTCTCGGAGAAGCCTGGTCCAGACCTCCACCCTTGAATAAAT 150
151 TACCTTTGTTGCTTTGGCAGGGCGCCTCGCGCCAGCGGCTTCGGCTGTTG 200
201 AGTACCTGCCAGAGGACCACAACTCTTGTTTTTAGTGATGTCTGAGTACT 250
251 ATATAATAGTTAAAACTTTCAACAACGGATCTCTTGGTTCTGGCATCGAT 300
301 GAAGAACGCAGCGAAATGCGATAAGTAATGTGAATTGCAGAATTCAGTGA 350
351 ATCATCGAATCTTTGAACGCACATTGCGCCCTCTGGTATTCCGGGGGGCA 400
401 TGCCTGTTCGAGCGTCATTATAACCACTCAAGCTCTCGCTTGGTATTGGG 450
451 GTTCGCGTCTTCGCGGCCTCTAAAATCAGTGGCGGTGCCTGTCGGCTCTA 500
501 CGCGTAGTAATACTCCTCGCGATTGAGTCCGGTAGGTTTACTTGCCAGCA 550
551 ACCCCCAATTTTTTACAGGTTGACCTCGGATCAGGTAGGGATACCCGCTG 606
601 AACTTAAGCATATCAATAAGCGGAGGA 650

The label extender probes and capture extender probes used are shown in Table 2. They were designed to be specific for the Rye-pathotype of *P. herpotrichoides.* The current probes and hybridisation conditions may allow the assay to cross-react with the Wheat-pathotype of *P. herpotrichoides* (98% gene conservation in the ITS region).

**Table 2:**

| Lable Extender and Capture Extender Probes | | | |
|---|---|---|---|
| 31-50 | LE1 | 317-337 | LE11 |
| 54-71 | CE1 | 341-361 | LE12 |
| 75-92 | LE2 | 365-382 | LE13 |
| 96-112 | LE3 | 386-402 | LE14 |
| 116-133 | LE4 | 406-426 | LE15 |
| 137-158 | CE2 | 429-447 | LE16 |
| 162-178 | LE5 | 451-467 | CE4 |
| 182-198 | LE6 | 471-489 | LE17 |
| 202-218 | LE7 | 493-514 | LE18 |
| 222-242 | CE3 | 518-535 | LE19 |
| 246-269 | LE8 | 539-556 | CE5 |
| 273-291 | LE9 | 560-579 | LE20 |
| 295-313 | LE10 | | |

The ITS region is a good gene region to target for assay development since this region is highly divergent at the species level. The more extensive sequence divergence in the ITS region has been the basis for the development of PCR assays that are able to detect plant pathogens including *Septoria, Mycosphaerella, P. herpotrichoides* and *Verticillium.*

Similar sets of probes may be developed to any known ITS sequence from any target pathogen of interest. These may include the wheat pathogens *Septoria nodorum, Septoria tritici, Septoria avenae f. sp. triticea, Fusarium* spp, *Microdochium nivale, Drechslera tritici-repentis* and *Rhizoctonia cerealis.* Since the ITS regions are known, assays may also be developed to the maize pathogens *Cercospora zea-maydis, Puccinia sorghi, Kabatiella zeae, Helminthosporium turcicum, Helminthosporium maydis* and *Helminthosporium carbonum.*

The assay characteristics are summarised as shown below.

| | |
|---|---|
| Sensing volume | 10 µl |
| Target concentration | 1.7 aM to 17 fM (1.7 x 10⁻²³ to 1.7 x 10⁻¹⁹ mole per 10µl) |
| Label extenders | 1 nM each (1 x 10⁻¹⁴ mole per 10µl of all 20 label extenders). |
| Capture extenders | 0.1 nM each (1 x 10⁻¹⁵ mole per 10µl of all 5 capture extenders). |
| Assay procedure | 10 min equilibration/washing, |
| | 30 min incubation (stopped flow), |
| | 10 min washing, |
| | 5 min regeneration, |
| | 5 min washing |
| Detection | 5 min after the incubation step during the washing step |
| Denaturation | 95 °C for 30 min |
| Sample rack | 1°C |

Net signal (average of 3 measurements): blank 4013±57 cps, 1.7 aM 3982±175 cps, 17 aM 4711±365, 170 aM 5721±237, 1.7 fM 11641±163, 17 fM 23509±1270 cps: LOD: 10 aM.

A universal capture probe (chip-3'-TTATAGTACTCCAATGCC-5') was immobilised via the 3' end to the chip surface by the following way:
**a) *Surface functionalization*:** liquid phase silanisation of the PWG-chips is carried out by treatment with 2 % (v/v) 3-glycidyloxypropyl trimethoxysilane and 0.2 % (v/v) N-ethyldiisopropylamin in o-xylene at elevated temperature (i.e. 50-90 °C) for several hours; silanised PWG-chips are cleaned in CH₃CN and dried in a desiccator under vacuum
***b) Immobilisation of Oligonucleotides:*** the surface bound epoxy rings react via ring opening with terminal amino or thiol groups of modified oligonucleotides: 100 µl of a solution containing 10 nmol of a thiol or amino terminated capture probe is incubated at 35 °C over night in 100 mM aqueous carbonate buffer, pH 8.2; chips with immobilised capture probes are rinsed with water, dried and stored at -20 °C until used in the PWG setup.

The R-pathotype genomic DNA target (10 - 10⁵ genomic equivalents) was mixed with a solution containing 5 capture extenders (CEs, 0.1 nM each) and 20 Cy5 labeled label extenders (LEs 1-20, 1 nM each) in a hybridisation buffer pH 7.0 (75 mM sodium citrate, 750 mM sodium chloride, 0.1% Tergitol NP-40). 200 µl of this mixture was heated to 95 °C for 30 min followed by storage in the sample rack at 1 °C for 30 min. The hybridisation assays were performed in the same hybridisation buffer at 53 °C. The sample mixture was incubated (using a stopped flow apparatus and method) for 30 min during the hybridisation assay. The lowest detected amount of target DNA was 100 genomic equivalents (in a volume of 10 µl) determined as the signal above three standard deviations of the signal determined for the negative control with three repetitions.

### Example 2: Pseudocercosporella herpotrichoides assay specificity: (sequence specific detection of the genomic DNA)

The target region on the genomic DNA is its ITS region, and the CE and LE probes are described in Example 1.

The specificity for the detection of the cereal pathogen of interest (*Pseudocersprolella herpotrichoides #308, R-pathotype*) was compared with the non-specific signal obtained by other cereal pathogen with the same concentration: *Microdochium nivale* #18222, *Fusarium graminearum* R-8417, *Septoria tritici* #26517, *Ceratobasidium cereale* (*Rhizoctonia cerealis*) #44234, *Drechslera sorokiniana* #11404, *Cercospora arachidicula* #52476 and *Septoria nodorum* #24425. At a target level of 10⁴ genomic equivalents of each cereal pathogen a specific, to non-specific signal ratio of 6.1 was observed in the assay for *Pseudocersprolella herpotrichoides #308 R-pathotype,* whereas a specific to non-specific signal ratio of 1.0 was determined for all other cereal pathogens.

The assay characteristics are summarised as shown below.

| | |
|---|---|
| Sensing volume | 10 µl |
| Target concentration | 1.7 fM (1.7 x 10⁻¹⁸ mole per 10µl) |
| Label extenders | 1 nM each (1 x 10⁻¹⁴ mole per 10µl of all 20 label extenders). |
| Capture extenders | 0.1 nM each (1 x 10⁻¹⁵ mole per 10µl of all 5 capture extenders). |
| Assay procedure | 10 min equilibration/washing, |
| | 30 min incubation (stopped flow), |
| | 10 min washing, |
| | 5 min regeneration, |
| | 5 min washing |
| Detection | 5 min after the incubation step during the washing step |
| Denaturation | 95 °C for 30 min |
| Sample rack | 1°C |
| Net signal (average of 3 measurements) | |
| blank pathogen of interest | |
| *Pseudocersprolella herpotrichoides* #308, R-pathotype | 7270±200 cps; |
| *Microdochium nivale* #18222 | 1136±206 cps; |
| *Fusarium graminearum* R-8417 | 1235±84 cps; |
| *Septoria tritici* #26517 | 1166±106 cps; |
| *Ceratobasidium cereale* (*Rhizoctonia cerealis*) #44234 | 1295±308 cps; |
| *Drechslera sorokiniana* #11404 | 1172±192 cps; |
| *Cercospora arachidicula* #52476 | 1091±115 cps |
| *Septoria nodorum* #24425 | 1020±63 cps. |

## Claims

1. A method of detecting a target polynucleotide employing two sets of reagents, a labelling set and a capture set, and wherein an optical planar waveguide is used, said method comprising
1) providing the labelling set of reagents, comprising
a) a plurality of label probes each comprising a label portion and a nucleic acid region (which is preferably single-stranded) having same or different sequences L1 (preferably between about 5 and 100 nucleotides (nt) long; more preferably between about 8 and 100 nt long) which are complementary to same or different sequences of the target polynucleotide, and optionally second sequences between the label portion and sequences L1, which are neither complementary to a sequence of the target polynucleotide nor to the capture extender or capture probes and which are less than about 500 nt long, and the label portion comprising a label which provides a signal which is detectable by luminescence, including evanescently excited luminescence, or
b) a plurality of (b1) label extender probes each comprising a label binding nucleic acid sequence L2 and nucleic acid sequences L1, wherein the sequencies L1 are complementary to nucleic acid sequences of the target polynucleotide and the sequence L2 is complementary to the nucleic acid sequence L3 of a label probe, and optionally (b2) a label probe comprising a nucleic acid sequence L3, which is complememtary to the the sequence of L2, and a label portion comprising a label which provides a signal which is detectable by luminescence, including evanescently excited luminescence, and
2) providing the capturing set of reagents, comprising
c) a plurality of capture probes comprising same or different nucleic acid sequences D 1 complementary to nucleic acid sequences of the target polynucleotide, wherein the nucleic acid sequences D1 (preferably between about 10 and 100 nt long) is not complementary to sequencies L1, L2 and L3 comprised in the label probe and the label extender probe and wherein the capture probe is bound or capable of being specifically bound to the surface the wave guiding layer of a waveguide, either directly or via linking groups; or
d) a plurality of (d1) capture extender probes each comprising a nucleic acid region (preferably single-stranded) having a sequence C1 (preferably between about 10 and 100 nt long) which is complementary to a sequence of the target polynucleotide and optionally a second region not being complementary to a sequence of the target polynucleotide and preferably being less than about 500 nt long, and further comprising a capture probe recognition nucleic acid sequence C2, and (d2) a capture probe and wherein the capture probe is bound or capable of being specifically bound to the surface the wave guiding layer of a waveguide, either directly or via linking groups, comprising nucleic acid sequences D2 complemetary to sequence C2, wherein sequences L1 and C1 are non-identical, non complementary sequences;
3) combining in an aqueous medium under binding conditions for complementary sequences, a sample in which the presence of the target polynucleotide is to be determined (which may have been treated so that the target polynucleotide is present in single-stranded form), with
a) compositions 1a) and 2c) or 1a) and 2d), or (b) compositions 1b) and 2c) or 1b) and 2d), or (c) compositions 1a), 1b) and 2c) or 1a), 1b) and 2d), or (d) compositions 1a), 2c) and 2d) or 1b), 2c) and 2d), ), or (e) compositions 1a), 1b), 2c) and 2d), so that complexes are formed;
4) binding the complexes of step 3 to the waveguide via a plurality of copies of the capture probe (the capture probe may be bound to the waveguide before binding to the complexes of step 3 or may be bound to the waveguide after binding to the complexes of step 3);
5) if the composition 1b) does not comprise a label probe and a label was not included in step 3 to label said complexes, then combining the bound complex of step 4 with the label;
6) detecting label bound to the target polynucleotide by measuring the luminescence that is excited in the evanescent field of the waveguide, or by measuring the luminescence that is generated in the near field of the waveguide,
whereby at least two label probes or label extenders, capture probes or capture extender probes, are used having different nucleic sequences which are complementary to different target nucleic acid sequences.

2. A method according to claim 1 wherein steps 3 and 5 are performed prior to step 4.

3. A method according to any one of claims 1 or 2 wherein the label portion comprises a luminescent compound.

4. A method of detecting a target polynucleotide according to claims 1 to 3, using a solution phase nucleic acid sandwich assay, wherein the solid support is an optical planar waveguide, and the label is detected by measuring the luminescence that is excited in the evanescent field of the waveguide, or the luminescence that is generated in the near field of the waveguide.

5. A method of detecting a target polynucleotide using an optical planar waveguide according to claims 1 to 4, wherein the target polynucleotide is immobilised on the waveguide by means of two classes of binding interactions, the first class of binding interaction being with at least one capture extender probe, and the second class of binding interaction being between at least one of said capture extender probes and at least one of multiple capture probes which are bound, or capable of being bound and are subsequently bound, to the surface of of the wave-guiding layer of the planar waveguide.

6. A method according to any one of claims 1 to 5 wherein the bound label is not separated from unbound label prior to measurement of bound label.

7. A method according to any one of claims 1-6 wherein a target polynucleotide is detected when less than 10⁴ copies of the target polynucleotide are present in sample volumes between 10 nl to 10 ml.

8. A method according to any one of claims 1 to 7 wherein less than 1000 equivalents of the genomic DNA in 10 µl of a sample volume.

9. A method according to any one of claims 1-8 wherein the capture probe is synthesised on the optical planar waveguide.

10. A method according to any one of claims 1-9 wherein more than one target polynucleotide is detected in a sample.

11. A method according to any one of claims 1-10 in which the target polynucleotide is a sequence from a pathogenic organism or is a polynucleotide comprising a sequence that is associated with a disease of a living organism.

12. A method according to claim 11 wherein the pathogenic organism is a virus.

13. Use of a method according to any one of claims 1-12 for the investigation or diagnosis of a disease associated with the target polynucleotide.

14. A kit of parts comprising capture probes according to 2c) of claim 1, capture extender probes according to 2d) of claim 1, label probes according to 1 a) of claim 1, and label extender probes together with label probes according to 1 b) of claim 1, whereby at least two label probes or label extender probes, capture probes or capture extender probes comprise different nucleic acid sequences which are complementary to different target nucleic acid sequences.

15. An optical planar waveguide wherein is bound to the surface of the wave -guiding layer of the planar waveguide
a) a plurality of capture probes as above defined under 2c), or a plurality of capture extender probes and a capture probe as above defined under 2d) of claim 1;
b) a target polynucleotide, bound to the capture probe or the capture extender probe; and
c) a plurality of lable probes as above defined under 1a) of claim 1, bound to the target polynucleotide, or a plurality of a lable probe extender as above defined under 1b) of claim 1, bound to the target polynucleotide, and a table probe as above defined under 1b) of claim 1, bound to said lable probe extender.

## Patentansprüche

1. Verfahren zum Nachweis eines Target-Polynukleotids, bei dem zwei Sets von Reagenzien, ein Labelling-Set und ein Capture-Set eingesetzt werden, und worin ein optischer planarer Wellenleiter verwendet wird, umfassend
1) die Bereitstellung des Labelling-Sets der Reagenzien, welches umfasst:
a) eine Vielzahl von Label-Proben, jeweils umfassend einen Label-Teil und einen Nukleinsäurebereich (der vorzugsweise einsträngig ist) mit gleichen oder verschiedenen Sequenzen L1 (vorzugsweise zwischen etwa 5 und 100 Nukleotiden (nt) lang; bevorzugter zwischen etwa 8 und 100 nt lang), die komplementär sind zu gleichen oder verschiedenen Sequenzen des Target-Polynukleotids, und gegebenenfalls zweite Sequenzen zwischen dem Label-Teil und Sequenzen L1, die weder komplementär sind zu einer Sequenz des Target-Polynukleotids noch zu dem Capture-Extender oder Capture-Proben und die weniger als etwa 500 nt lang sind, wobei der Label-Teil ein Label umfasst, das ein Signal liefert, welches nachweisbar ist durch Lumineszenz einschließlich evaneszent angeregter Lumineszenz, oder
b) eine Vielzahl von (b1) Label-Extender-Proben, von denen eine jede eine Labelbindende Nukleinsäuresequenz L2 und Nukleinsäuresequenzen L1 umfasst, worin die Sequenzen L1 komplementär sind zu Nukleinsäuresequenzen des Taraet-Polynukleotids und die Sequenz L2 komplementär ist zu der Nukleinsäuresequenz L3 einer Label-Probe, und gegebenenfalls (b2) eine Label-Probe, umfassend eine Nukleinsäuresequenz L3, die komplementär ist zu der Sequenz L2, und ein Label-Teil, umfassend ein Label, das ein Signal liefert, welches nachweisbar ist durch Lumineszenz einschließlich der evaneszent angeregten Lumineszenz, und
2) die Bereitstellung des Capture-Sets von Reagenzien, umfassend
c) eine Vielzahl von Capture-Proben, umfassend gleiche oder verschiedene Nukleinsäuresequenzen D1, die komplementär sind zu Nukleinsäuresequenzen des Target-Polynukleotids, worin die Nukleinsäuresequenzen D1 (vorzugsweise zwischen etwa 10 und 100 nt lang) nicht komplementär sind zu Sequenzen L1, L2 und L3, die in der Label-Probe und der Label-Extender-Probe enthalten sind, und worin die Capture-Probe gebunden ist an oder befähigt ist, spezifisch gebunden zu werden an die Oberfläche der Wellenleiterschicht eines Wellenleiters, entweder direkt oder über verknüpfende Gruppen; oder
d) eine Vielzahl von (d1) Capture-Extender-Proben, jeweils umfassend einen Nukleinsäurebereich (vorzugsweise einsträngig) mit einer Sequenz C1 (vorzugsweise zwischen etwa 10 und 100 nt lang), die komplementär ist zu einer Sequenz des Target-Polynukleotids, und gegebenenfalls einen zweiten Bereich, der nicht komplementär ist zu einer Sequenz des Target-Polynukleotids und vorzugsweise kleiner ist als etwa 500 nt lang, und weiterhin umfassend eine Capture-Probenerkennungs-Nukleinsäuresequenz C2, und (d2) eine Capture-Probe und worin die Capture-Probe gebunden ist an oder befähigt ist, gebunden zu werden an die Oberfläche der Wellenleiterschicht eines Wellenleiters entweder direkt oder über verknüpfende Gruppen, umfassend Nukleinsäuresequenzen D2, die komplementär sind zur Sequenz C2, worin die Sequenzen L1 und C1 nicht-identische, nicht-komplementäre Sequenzen sind;
3) Kombinieren in einem wässrigen Medium unter bindenden Bedingungen für komplementäre Sequenzen, einer Testprobe, worin die Anwesenheit des Target-Polynukleotids bestimmt werden soll (das derart behandelt worden sein kann, dass das Target-Polynukleotid in einsträngiger Form vorliegt), mit
a) Zusammensetzungen 1a) und 2c) oder 1a) und 2d), oder (b) Zusammensetzungen 1b) und 2c) oder 1b) und 2d), oder (c) Zusammensetzungen 1a), 1b) und 2c) oder 1a), 1b) und 2d), oder (d) Zusammensetzungen 1a), 2c) und 2d) oder 1b), 2c) und 2d), oder (e) Zusammensetzungen 1a), 1b), 2c) und 2d), so dass Komplexe gebildet werden;
4) Binden der Komplexe von Stufe 3 an den Wellenleiter über eine Vielzahl von Kopien der Capture-Probe (die Capture-Probe kann an den Wellenleiter vor dem Binden an die Komplexe von Stufe 3 gebunden werden oder an den Wellenleiter nach Binden an die Komplexe von Stufe 3 gebunden werden);
5) wenn die Zusammensetzung 1b) keine Label-Probe umfasst und ein Label nicht in Stufe 3 eingeschlossen ist, um besagte Komplexe einem Labelling zu unterziehen, dann Kombinieren des gebundenen Komplexes von Stufe 4 mit dem Label;
6) Nachweis des an das Target-Polynukleotid gebundenen Labels durch Messen der Lumineszenz, die in dem evaneszenten Feld des Wellenleiters angeregt wird, oder durch Messen der Lumineszenz, die in dem nahen Feld des Wellenleiters erzeugt wird,
wobei zumindest zwei Label-Proben oder Label-Extender, Capture-Proben oder Capture-Extender-Proben mit verschiedenen Nukleinsäuresequenzen, die zu verschiedenen Target-Nukleinsäuresequenzen komplementär sind, verwendet werden.

2. Verfahren gemäß Anspruch 1, worin die Stufen 3 und 5 vor Stufe 4 durchgeführt werden.

3. Verfahren gemäß einem der Ansprüche 1 oder 2, worin das Label-Teil eine lumineszierende Verbindung umfasst.

4. Verfahren zum Nachweis eines Target-Polynukleotids gemäß den Ansprüchen 1 bis 3, unter Verwendung eines Nukleinsäure-Sandwich-Assays mit Lösungsphase, worin der feste Träger ein optischer planarer Wellenleiter ist und das Label nachgewiesen wird durch Messen der Lumineszenz, die in dem evaneszenten Feld des Wellenleiters angeregt wird, oder Lumineszenz, die in dem nahen Feld des Wellenleiters erzeugt wird.

5. Verfahren zum Nachweis eines Target-Polynukleotids unter Verwendung eines optischen planaren Wellenleiters gemäß den Ansprüchen 1 bis 4, worin das Target-Polynukleotid auf dem Wellenleiter immobilisiert wird mit Hilfe von zwei Klassen von Bindungs-Wechselwirkungen, wobei die erste Klasse der Bindungs-Wechselwirkung mit zumindest einer Capture-Extender-Probe stattfindet und die zweite Klasse der Bindungs-Wechselwirkung stattfindet zwischen zumindest einer der besagten Capture-Extender-Proben und zumindest einer der zahlreichen Capture-Proben, die gebunden sind an oder befähigt sind, gebunden zu werden und anschließend gebunden werden an die Oberfläche der Wellenleiterschicht des planaren Wellenleiters.

6. Verfahren gemäß einem der Ansprüche 1 bis 5, worin das gebundene Label von dem nicht-gebundenen Label vor der Messung des gebundenen Labels nicht getrennt wird.

7. Verfahren gemäß einem der Ansprüche 1 bis 6, worin ein Target-Polynukleotid nachgewiesen wird, wenn weniger als 10⁴ Kopien des Target-Polynukleotids in Testprobenvolumina zwischen 10 nl bis 10 ml vorhanden sind.

8. Verfahren gemäß einem der Ansprüche 1 bis 7, worin weniger als 1 000 Äquivalente der genomen DNA in 10 µl eines Testprobenvolumens vorliegen.

9. Verfahren gemäß einem der Ansprüche 1 bis 8, worin die Capture-Probe auf dem optischen planaren Wellenleiter synthetisiert wird.

10. Verfahren gemäß einem der Ansprüche 1 bis 9, worin mehr als ein Target-Polynukleotid in einer Testprobe nachgewiesen wird.

11. Verfahren gemäß einem der Ansprüche 1 bis 10, worin das Target-Polynukleotid eine Sequenz aus einem pathogenen Organismus ist oder ein Polynukleotid ist, das eine Sequenz umfasst, die mit einer Erkrankung eines lebenden Organismus assoziiert ist.

12. Verfahren gemäß Anspruch 11, worin der pathogene Organismus ein Virus ist.

13. Verwendung eines Verfahren gemäß einem der Ansprüche 1 bis 12 für die Untersuchung oder Diagnose einer Erkrankung, die mit dem Target-Polynukleotid assoziiert ist.

14. Kit aus Teilen, umfassend Capture-Proben gemäß 2c) von Anspruch 1, Capture-Extender-Proben gemäß 2d) von Anspruch 1, Label-Proben gemäß 1a) von Anspruch 1, und Label-Extender-Proben zusammen mit Label-Proben gemäß 1b) von Anspruch 1, wobei zumindest zwei Label-Proben oder Label-Extender-Proben, Capture-Proben oder Capture-Extender-Proben verschiedene Nukleinsäuresequenzen umfassen, die komplementär sind zu verschiedenen Target-Nukleinsäuresequenzen.

15. Optischer planarer Wellenleiter, worin an die Oberfläche der Wellenleiterschicht des planaren Wellenleiters gebunden ist:
a) eine Vielzahl von Capture-Proben, wie unter 2c) definiert, oder eine Vielzahl von Capture-Extender-Proben und einer Capture-Probe, wie unter 2d) von Anspruch 1 definiert;
b) ein Target-Polynukleotid, das gebunden ist an die Capture-Probe oder die Capture-Extender-Probe; und
c) eine Vielzahl von Label-Proben, wie unter 1a) von Anspruch 1 definiert, die gebunden sind an das Target-Polynukleotid, oder eine Vielzahl von Label-Proben-Extendern, wie unter 1b) von Anspruch 1 definiert, die gebunden sind an das Target-Polynukleotid, und eine Label-Probe, wie unter 1b) von Anspruch 1 definiert, die an den besagten Label-Proben-Extender gebunden ist.

## Revendications

1. Procédé pour détecter un polynucléotide cible employant deux jeux de réactifs, un jeu de marquage et un jeu de capture, et dans lequel un guide d'onde optique plan est utilisé, ledit procédé comprenant les étapes consistant à :
1) fournir le jeu de réactifs de marquage, comprenant :
a) une pluralité de sondes de marquage comprenant chacune une partie de marquage et une région d'acides nucléiques (qui est de préférence mono-brin) ayant des séquences L1 identiques ou différentes (de préférence d'une longueur comprise entre environ 5 et 100 nucléotides (nt) ; de façon plus particulièrement préférée d'une longueur comprise entre environ 8 et 100 nt) qui sont complémentaires aux séquences identiques ou différentes du polynucléotide cible, et éventuellement des deuxièmes séquences entre la partie de marquage et les séquences L1, qui ne sont ni complémentaires à une séquence du polynucléotide cible, ni aux sondes de capture ou d'extension de capture et qui ont une longueur inférieure à environ 500 nt, et la partie de marquage comprenant une marque qui fournit un signal qui est détectable par luminescence, incluant une luminescence excitée de façon évanescente ; ou
b) une pluralité de (b1) sondes d'extension de marquage comprenant chacune une séquence d'acides nucléiques L2 liant une marque et des séquences d'acides nucléiques L1, dans lequel les séquences L1 sont complémentaires aux séquences d'acides nucléiques du polynucléotide cible et la séquence L2 est complémentaire à la séquence d'acides nucléiques L3 d'une sonde de marquage, et éventuellement (b2) une sonde de marquage comprenant une séquence d'acides nucléiques L3, qui est complémentaire à la séquence de L2, et une partie de marquage comprenant une marque qui fournit un signal qui est détectable par luminescence, incluant une luminescence excitée de façon évanescente ; et
2) fournir le jeu de réactifs de capture, comprenant :
c) une pluralité de sondes de capture comprenant des séquences d'acides nucléiques D1 identiques ou différentes, complémentaires aux séquences d'acides nucléiques du polynucléotide cible, dans lequel les séquences d'acides nucléiques D1 (de préférence d'une longueur comprise entre environ 10 et 100 nt) ne sont pas complémentaires aux séquences L1, L2 et L3 comprises dans la sonde de marquage et la sonde d'extension de marquage et dans lequel la sonde de capture est liée ou capable d'être spécifiquement liée à la surface de la couche de guidage d'onde d'un guide d'onde, soit directement, soit par l'intermédiaire de groupes de liaison ; ou
d) une pluralité de (d1) sondes d'extension de capture comprenant chacune une région d'acide nucléique (de préférence mono-brin) ayant une séquence C1 (de préférence d'une longueur comprise entre environ 10 et 100 nt) qui est complémentaire à une séquence du polynucléotide cible et éventuellement une deuxième région n'étant pas complémentaire à une séquence du polynucléotide cible et de préférence étant d'une longueur inférieure à environ 500 nt, et comprenant de plus une séquence de reconnaissance de sonde de capture d'acide nucléique C2, et (d2) une sonde de capture et dans lequel la sonde de capture est liée ou capable d'être spécifiquement liée à la surface de la couche de guidage d'onde d'un guide d'onde, soit directement, soit par l'intermédiaire de groupes de liaison, comprenant des séquences d'acides nucléiques D2 complémentaires à la séquence C2, dans lequel les séquences L1 et C1 sont des séquences non identiques, non complémentaires ;
3) combiner, dans un milieu aqueux, dans des conditions de liaison pour des séquences complémentaires, un échantillon dans lequel la présence du polynucléotide cible doit être déterminée (qui peut avoir été traité de sorte que le polynucléotide cible est présent sous forme mono-brin), avec
a) des compositions 1a) et 2c) ou 1a) et 2d), ou (b) des compositions 1b) et 2c) ou 1b) et 2d), ou (c) des compositions 1a), 1b) et 2c) ou 1a), 1b) et 2d), ou (d) des compositions 1a), 2c) et 2d) ou 1b), 2c) et 2d),), ou (e) des compositions 1a), 1b), 2c) et 2d), de sorte que des complexes sont formés ;
4) lier les complexes de l'étape 3 au guide d'onde par l'intermédiaire d'une pluralité de copies de la sonde de capture (la sonde de capture peut être liée au guide d'onde avant d'être liée aux complexes de l'étape 3 ou peut être liée au guide d'onde après avoir été liée aux complexes de l'étape 3) ;
5) si la composition 1b) ne comprend pas de sonde de marquage et qu'une marque n'était pas comprise dans l'étape 3 pour marquer lesdits complexes, combiner alors le complexe lié de l'étape 4 avec la marque ;
6) détecter la marque liée au polynucléotide cible en mesurant la luminescence qui est excitée dans le champ d'évanescence du guide d'onde, ou en mesurant la luminescence qui est engendrée dans le champ proche du guide d'onde ;
ainsi au moins deux sondes de marquage ou d'extension de marquage, des sondes de capture ou des sondes d'extension de capture, sont utilisées en ayant des séquences d'acides nucléiques différentes qui sont complémentaires aux séquences cibles différentes d'acides nucléiques.

2. Procédé selon la revendication 1, dans lequel les étapes 3 et 5 sont réalisées avant l'étape 4.

3. Procédé selon l'une quelconque des revendications 1 ou 2, dans lequel la partie de marquage comprend un composé luminescent.

4. Procédé pour détecter un polynucléotide cible selon les revendications 1 à 3, utilisant un dosage sandwich d'acides nucléiques en solution, dans lequel le support solide est un guide d'onde optique plan, et la marque est détectée en mesurant la luminescence qui est excitée dans le champ d'évanescence du guide d'onde, ou la luminescence qui est engendrée dans le champ proche du guide d'onde.

5. Procédé pour détecter un polynucléotide cible utilisant un guide d'onde optique plan selon les revendications 1 à 4, dans lequel le polynucléotide cible est immobilisé sur le guide d'onde au moyen de deux classes d'interactions de liaison, la première classe d'interaction de liaison étant avec au moins une sonde d'extension de capture, et la deuxième classe d'interaction de liaison étant entre au moins une desdites sondes d'extension de capture et au moins une des multiples sondes de capture qui sont liées, ou capables d'être liées et sont ultérieurement liées, à la surface de la couche de guidage d'ondes du guide d'onde plan.

6. Procédé selon l'une quelconque des revendications 1 à 5, dans lequel la marque liée n'est pas séparée de la marque non liée avant la mesure de la marque liée.

7. Procédé selon l'une quelconque des revendications 1 à 6, dans lequel un polynucléotide cible est détecté quand moins de 10⁴ copies du polynucléotide cible sont présentes dans des volumes échantillons compris entre 10 nL et 10 mL.

8. Procédé selon l'une quelconque des revendications 1 à 7, dans lequel il y a moins de 1 000 équivalents de l'ADN génomique dans un volume de 10 µL d'échantillon.

9. Procédé selon l'une quelconque des revendications 1 à 8, dans lequel la sonde de capture est synthétisée sur le guide d'onde optique plan.

10. Procédé selon l'une quelconque des revendications 1 à 9, dans lequel plus d'un polynucléotide cible est détecté dans un échantillon.

11. Procédé selon l'une quelconque des revendications 1 à 10, dans lequel le polynucléotide cible est une séquence provenant d'un organisme pathogène ou est un polynucléotide comprenant une séquence qui est associée à une maladie d'un organisme vivant.

12. Procédé selon la revendication 11, dans lequel l'organisme pathogène est un virus.

13. Utilisation du procédé selon l'une quelconque des revendications 1 à 12, pour l'étude ou le diagnostic d'une maladie associée au polynucléotide cible.

14. Trousse de pièces comprenant des sondes de capture selon 2c) de la revendication 1, des sondes d'extension de capture selon 2d) de la revendication 1, des sondes de marquage selon 1a) de la revendication 1, et des sondes d'extension de marquage conjointement avec des sondes de marquage selon 1b) de la revendication 1, ainsi au moins deux sondes de marquage ou des sondes d'extension de marquage, des sondes de capture ou des sondes d'extension de capture comprennent des séquences d'acides nucléiques différentes qui sont complémentaires aux différentes séquences cibles d'acides nucléiques.

15. Guide d'onde optique plan dans lequel est lié à la surface de la couche de guidage d'ondes du guide d'onde plan :
a) une pluralité de sondes de capture telles que définies ci-dessus dans 2c), ou une pluralité de sondes d'extension de capture et une sonde de capture telles que définies ci-dessus dans 2d) de la revendication 1;
b )un polynucléotide cible, lié à la sonde de capture ou à la sonde d'extension de capture ; et
c ) une pluralité de sondes de marquage telles que définies ci-dessus dans 1a) de la revendication 1, liées au polynucléotide cible, ou une pluralité de sondes d'extension de marquage telles que définies ci-dessus dans 1b) de la revendication 1, liées au polynucléotide cible, et une sonde de marquage telle que définie ci-dessus dans 1b) de la revendication 1, liée à ladite sonde d'extension de marquage.
